**Europäisches Patentamt**

**European Patent Office**

**Office européen des brevets**

(19)

(11) Publication number : **0 448 146 B1**

## (12) EUROPEAN PATENT SPECIFICATION

(45) Date of publication of patent specification :
**15.06.94 Bulletin 94/24**

(51) Int. Cl.⁵ : **A61K 47/12,** A61K 47/26,
A61K 37/38

(21) Application number : **91200483.5**

(22) Date of filing : **06.03.91**

(54) **Stabilized gonadotropin containing preparations.**

(30) Priority : **20.03.90 EP 90200665**

(43) Date of publication of application :
**25.09.91 Bulletin 91/39**

(45) Publication of the grant of the patent :
**15.06.94 Bulletin 94/24**

(84) Designated Contracting States :
**AT BE CH DE DK ES FR GB GR IT LI NL SE**

(56) References cited :
**EP-A- 0 124 018**
**EP-A- 0 302 772**
**WO-A-87/05300**
**FR-A- 2 300 573**
**US-A- 3 932 943**

(73) Proprietor : **AKZO N.V.**
**Velperweg 76**
**NL-6824 BM Arnhem (NL)**

(72) Inventor : **De Meere, Andreas Leo Johannes**
**Vrouwkensvaartsestraat 11**
**NL-5165 NN Waspik (NL)**
Inventor : **De Ruiter, Marinus Arie**
**Middenkamp 7**
**NL-5345 GM Oss (NL)**

(74) Representative : **Hermans, Franciscus G.M. et
al**
**Patent Department**
**AKZO NOBEL N.V.**
**Pharma Division**
**P.O. Box 20**
**NL-5340 BH Oss (NL)**

EP 0 448 146 B1

## Description

This invention relates to pharmaceutical compositions generally, and to stabilized gonadotropin containing preparations specifically.

Relatively pure gonadotropin preparations are commercially available. For example, compositions containing naturally derived human menopausal gonadotropin ("HMG") and naturally derived human chorionic gonadotropin ("HCG") are available as freeze-dried preparations under the trade designations "Humegon" and "Pregnyl," respectively, from Organon International, bv of Oss, NL. Pregnant mare gonadotropin is also available in a freeze dried form from the same company.

A bulking agent, e.g. mannitol, is added to these preparations before lyophilization. They do not require the addition of a stabilizer to ensure an adequate shelf-life. Evidently whatever natural contaminants remain after the purification process act to stabilize the preparations in freeze-dried form.

Recently however, with the advent of more effective production and purification techniques, preparations of certain very pure gonadotropins are insufficiently stable. They degrade in a relatively short time, losing activity. In order to prevent or slow down this degradation, attempts were made to freeze-dry (lyophilize) the preparations. Lyophilization has only been partially successful however.

A need exists for a gonadotropin - containing pharmaceutical preparation which is stable over a sufficiently long period of time for the product to be manufactured, shipped, and stored prior to use. The need is especially great for a stable preparation containing more than one gonadotropin.

In US Patent 3,932,943 a method of preparation of lyophilized serum or plasma is disclosed, in which citric acid is added prior to freeze-drying. The added acid serves to control the pH of the lyophilized products upon dissolution. European Patent Application 0,302,772 discloses a powdered composition for nasal administration of a physiologically active peptide, which also contains a water-soluble organic acid, like glutamic acid or succinic acid. The organic acid in these compositions serve as an absorption promotor for the active peptide. The present invention uses acid salts as stabilizer for lyophilized gonadotropins.

Generally, the invention includes a gonadotropin containing lyophilized protein preparation which contains a dicarboxylic acid salt stabilizer. "Dicarboxylic acid," as used herein, means an organic acid having two or more carboxylic acid moieties (e.g HOOC-R-COOH). The gonadotropin will be in admixture with, and at least partially capable of stabilization by, the particular stabilizer in lyophilized systems. The preparation will contain a sufficient amount of dicarboxylic acid salt to stabilize the gonadotropin in its freeze-dried form for a desired time at a desired temperature.

Typical dicarboxylic acid salts are salts of citric acid, tartaric acid, aspartic acid, or mixtures of these acids. The gonadotropin or gonadotropin derivatives (as used herein "gonadotropins") will typically be proteins such as follicle stimulating hormone (FSH), thyroid stimulating hormone (TSH), human chorionic gonadotropin (HCG), or luteinizing hormone (LH). The preparation can further include a non-reducing disaccharide, such as sucrose or trehalose.

The invention also includes a method of stabilizing an essentially pure gonadotropin, in lyophilized form, which method involves mixing the gonadotropin, in solution, with a sufficient amount of a dicarboxylic acid salt to stabilize the protein in the lyophilized form, and then freeze-drying the resulting solution to form a stabilized lyophilisate of the gonadotropin.

The invention further includes the reconstituted injectable preparation made from the lyophilisate. The injectable preparation consists essentially of aqueous solution of water for injection, the gonadotropin, a non-reducing sugar, an anti-adsorption agent, and the dicarboxylic acid salt.

FIG. 1 is a graph depicting the correlation between ionic strength (X 1000) versus the amount of recovery of recombinant FSH activity after 1 month at 60° C using various stabilizers.

FIG. 2 is a graph depicting the effect of citrate/protein ratio (weight/weight) on recovery of HCG activity.

## A. Gonadotropins-

Preferred gonadotropins are FSH, TSH, HCG, LH, derivatives and mixtures thereof, with or without other protein components. Follicle stimulating hormone, thyroid stimulating hormone, human chorionic gonadotropin, and luteinizing hormone are all chemically related proteins which consist of $\alpha$ and $\beta$ subunits. The $\alpha$ subunits of these proteins are identical or nearly so.

Follicle stimulating hormone is a hormonal glycoprotein of the anterior pituitary required for normal reproductive function. Follicle stimulating hormone has been used to stimulate development of ovarian follicles for in vitro fertilization, and has also been used clinically to stimulate follicular maturation in anovulatory women with chronic anovulatory syndrome or luteal phase deficiency. Follicle stimulating hormone may be at least partially isolated from natural sources, such as human urine. Recombinant follicle stimulating hormone and/or

2

LH may be prepared as described in Keene et al "Expression of Biologically Active Human Follitropin in Chinese Hamster Ovary Cells," The Journal of Biological Chemistry, Vol. 264, pp. 4769-4775 (25 March 1989), the contents of which are incorporated by this reference. As used herein, a gonadotrophin, for example follicle stimulating hormone (FSH), includes the compound's analogs, and its recombinant, natural, deglycosylated, unglycosylated, modified glycosylated, and other forms.

The most preferred gonadotropin is FSH produced by recombinant DNA techniques (rFSH), either alone or in a lyophilisate with LH or HCG. FSH purified from natural sources is generally only partially purified. The impurities seem to act to stabilize it somewhat. With rFSH, however the impurities are not present, and thus the FSH is more susceptible to rapid degradation and freeze-drying losses. Doses of FSH range from 60 to 1500, especially 75 to 225 IU per ampoule lyophilisate.

Any gonadotropin used is preferably present in the lyophilisate preparations in a quantity sufficient to form a therapeutically useful concentration of the protein after dilution, generally for parenteral (e.g. subcutaneous or intravenous) administration, with a specified amount of an aqueous solution (e.g. distilled water for injection or sterile normal saline) to form a volume of preparation contemplated for use. As used herein, an aqueous solution is a solution containing water as a primary, but not necessarily the only, solvent. For example, a container containing FSH may contain 1 to 1000 micrograms ($\mu$g) of FSH (e.g. 75 international units is considered a therapeutic amount). Preferably, the highest reasonable amount of protein possible will be present in a container, since the greater the amount of protein present, generally the more stable the preparation. Useful doses of gonadotropins are known to medical practitioners, and the amount included in a dose is generally dependent upon the disease state and the particular patient being treated.

Illustratively, amounts as high as 10,000 international units and as low as 15 international units of HCG have been administered. Injections ranging from 20 to 225 international units LH have been used.

In one preferred embodiment, a combination of FSH and LH or FSH and HCG are lyophilised together to from a preparation having therapeutic amounts of both of the selected gonadotropins.

B. Stabilizers-

As used herein, "stabilize" is a relative term. To stabilize with a stabilizing agent or compound means the ability to prevent or delay a decrease in the protein's activity with the stabilizing agent. For example, a preparation would be deemed "stabilized" if, with the addition of a stabilizing compound ("stabilizer"), it took longer (e.g. 2 weeks instead of 1 week) to degrade at a set temperature, thus losing some of its in vivo or in vitro activity in comparison to the preparation sans the stabilizer.

A protein's activity may be determined by known methods relating to the particular protein. One measure of activity can be made by measuring the amount of (inactive) oligomers formed over time. Oligomer formation in a sample can be determined by HPSEC.

Other methods of determining the residual activity of, for example, rFSH include enzyme immunoactivity assay ("EIA") as described in U.S. Patent Reissue No. 32,696 to Schuurs et al; a kit available under the trade designation "FSHEIA" from bioMérieux of Marcy l'Etoile 69260 Charbonnières-les-Bains, France for FSH; and in vitro bioassay of both FSH and LH as described in Mannaerts et al, "Applications of in vitro Bioassays for Gonadotropins," Neuroendocrinology of Reproduction, pp. 49 - 58 (Elsevier Science Publishers bv, Amsterdam, NL 1987).

Preferred stabilizers for use with the preparations are salts of dicarboxylic acids such as citric acid, tartaric acid, aspartic acid, and mixtures thereof. Preferred salts are the sodium, potassium, lithium and ammonium salts of such dicarboxylic acids, especially sodium and potassium salts. Another dicarboxylic acid salt is sodium glutamate. The presence of a dicarboxylic acid salt stabilizer acts to stabilize the enzymatic mixtures, especially at relatively higher temperatures over longer periods of time.

When used as stabilizers, aspartate and glutamate salts gave better recovery of activity than did citrate, isocitrate, or tartrate salts.

When a citrate salt is the selected stabilizer, a ratio of citrate to gonadotropin in the range of 200 to 400 (mg/mg) is preferred, giving the best stabilization and recovery in the presence of some sugar. An especially preferred protein for use with sodium citrate is rFSH, due to the compound's ability to be stabilized with the stabilizer.

Concentrations of dicarboxylic acid salt stabilizers sufficient to form a solution having an ionic strength of greater than 0.050 are preferred in FSH compositions containing between 0.1 and 1000 $\mu$g of FSH. Especially preferred are those solutions having an ionic strength of between 0.250 and 0.350 which will generally stabilize, for example, rFSH stored at one month at 60°C to yield a 75% recovery of rFSH. Calculation of ionic strength is well-known to those skilled in the art, for example see Chase, et al, Remington's Pharmaceutical Sciences, pp. 223-224, 228, and 233 (16th ed. 1980, Mack Publ. Co. of Easton, PA, U.S.A.).

Concentrations of 2.5 to 17.5 milligrams per milliliter (mg/ml) of sodium citrate in solution are generally sufficient to stabilize lyophilized rFSH in the amounts described herein.

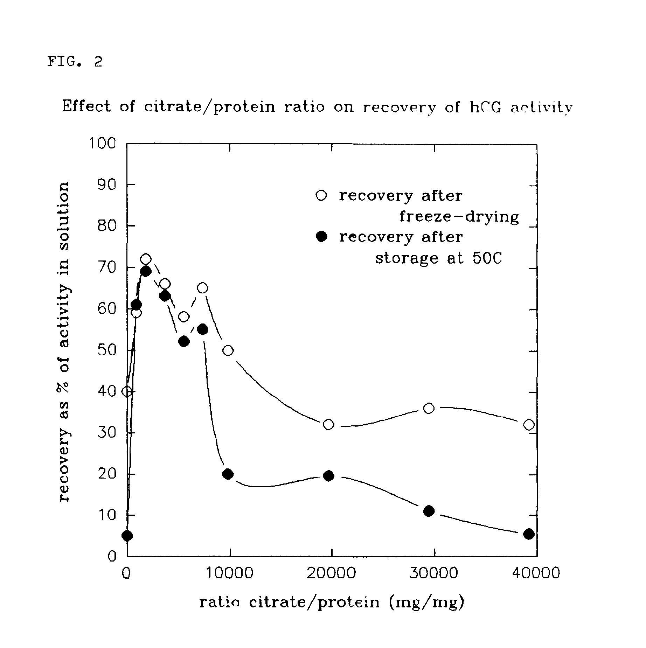

Also as depicted in FIG. 2, citrate should be present in a composition with, for example, HCG in an amount less than 10,000 times by weight that of the HCG to achieve greater stability.

## C. Non-reducing Sugars-

The compositions to be freeze-dried preferably contain a non-reducing sugar such as sucrose or trehalose. The incorporation of such a sugar, e.g. sucrose, acts to increase the "collapse (or 'shrinkage') temperature" at which the lyophilization of the solution takes place. This increase in temperature simplifies the entire freeze-drying process. An especially preferred non-reducing sugar for this use is sucrose in its amorphic state.

The amount of non-reducing sugar present in the solution to be lyophilized will generally be dependent upon the amount of dicarboxylic acid salt stabilizer present. For example, the weight ratio of non-reducing sugar to dicarboxylic acid salt will generally vary between 50 : 1 to 10 : 3 with a preferred concentration being about 3.3 : 1 in the case of sucrose to sodium citrate. Especially preferred is a solution containing 50 mg/ml sucrose and 14.7 mg/ml sodium citrate which also yields an optimal lyophilisate in terms of physical characteristics.

In the presently most preferred embodiments, the amount of sucrose will be sufficient to raise the collapse temperature from -38° C to about -25°C as determined by differential scanning calorimetry. The resulting lyophilisate "cake" remains amorphous and stable for relatively longer periods of time.

## D. Anti-absorption agents-

Anti-adsorption agents are preferably added to the lyophilized composition to prevent adsorbance of the protein to the walls of the container in which the compositions are contained, thus preventing a possible decrease in concentration. Certain anti-adsorption agents (e.g. polysorbates) also act as "cryoprotectants" protecting the protein during the lyophilization process.

Preferred anti-adsorption agents are nonionic surfactants such as Polysorbate 20, NF (Tween® 20 available from Atlas Chemical Company), Polysorbate 80, NF (Tween® 80 available from Atlas Chemical Company), Brij® 35 (available from ICI Pharmaceuticals of Great Britain), and Pluronic® F123 (available from BASF of Ludwigshafen, W. Germany). Polysorbate 20, NF is especially preferred.

Polysorbate is preferably understood as meaning a polysorbate which meets the specification of USP/NF XXII, which is published as "The National Formulary", p. 1763 and 1967, Official from 1 Jan. 1990 (22nd ed., U.S. Pharmacopeial Convention, Inc. 1989).

An anti-adsorption agent or anti-adsorption agents will be present in such amounts that adsorption of the protein onto container walls, or walls of vessels during processing, is decreased. Illustratively, amounts of Polysorbate 20 sufficient to form a concentration between 0.1 and 0.2 mg / ml in the ultimate solution for use are preferred. Concentrations higher than this tend to lead to oligomer formation, and thus decreased activity.

## E. Pharmaceutical Compositions-

The stable lyophilized preparation of the instant invention can be prepared by admixing the selected protein in aqueous solution with a sufficient amount of a dicarboxylic acid salt stabilizer to stabilize the protein, and a sufficient amount of a non-reducing sugar to increase the collapse temperature from -38°C to greater than -25°C. Temperatures greater than -35°C are preferred. Optionally, the selected anti-adsorption agent may also be added. The solution is then filtered, placed into containers (e.g. one ml glass ampoules) and then freeze-dried to form a stabilized lyophilisate. Freeze-drying techniques are well-known to those of skill in the art. For more information, reference may be made to several texts, including Goldblith et al, Freeze Drying and Advanced Food Technology, (Academic Press, Inc., London, GB 1975). Preferred residual water content in the lyophilisate cakes are between 1 and 5 %. Aseptic techniques should be used throughout the procedure. Freeze-driers are available from manufacturers such as Leybold or Edwards. Using such a procedure, or modifications thereof, several different compositions may be prepared.

An especially preferred composition contains rFSH in admixture with a stabilizer which is a salt of a dicarboxylic acid, wherein the dicarboxylic acid is selected from the group consisting of citric acid, tartaric acid, aspartic acid, and mixtures of these acids.

Another preferred lyophilized preparation contains, in admixture, a dicarboxylic acid salt stabilizer, a gonadotropin capable of stabilization by the amount of stabilizer present in the preparation, and trehalose. This preparation further include sodium biphosphate in admixture with the stabilizer, protein, and non-reducing sugar. Especially preferred salts for such preparations are sodium aspartate, sodium citrate, and sodium tartrate.

Another preferred stable lyophilized preparation contains, in admixture, a stabilizer such as a salt of tartaric or aspartic acid, a gonadotropin capable of stabilization by the amount of stabilizer present in the preparation, and a non-reducing sugar. The preparation may further include disodium biphosphate in admixture with the stabilizer, protein, and non-reducing sugar. Especially preferred non-reducing sugars are trehalose and sucrose. An especially preferred stabilizer in such preparations is sodium aspartate.

Another highly preferred stabilized lyophilisate consists essentially of a protein; a sufficient amount of a dicarboxylic acid salt stabilizer to stabilize the protein in freeze dried form; a disaccharidic non-reducing sugar; an anti-adsorption agent to prevent said protein from adsorbing onto a container containing the lyophilisate; and less than five percent residual water. In such a lyophilisate the protein will be FSH; the dicarboxylic acid salt stabilizer will be selected from the group consisting of salts of citric acid, tartaric acid, and aspartic acid; the disaccharidic non-reducing sugar will either be sucrose or trehalose, and the anti-adsorption agent will be selected from the group consisting of Tween® 20, Tween® 80, Brij®, or pluronic acid. This lyophilisate is especially preferred since, among other things, it has been discovered that the addition of further "stabilizers," such as mannitol, maltose, or either of them actually act to destabilize the lyophilisate in terms of activity.

Methods for making parenteral preparations and intravenous admixtures are disclosed in Remington's Pharmaceutical Sciences, pp. 1463-1497. However, caution must be exercised since although the stabilized compositions are compatible with infusion liquids, the infusion liquid used preferably should not contain reducing sugars. The preferred pH of the resulting solution for use should be between 6 and 8, especially 7.

The invention is further explained by reference to the following EXAMPLES:

## EXAMPLE I

### A. Stabilization of rFSH utilizing various disaccharides.

Aqueous solutions containing 150 units of rFSH were prepared. The solutions were divided into three groups and each group was mixed with (1) 50 mg/ml maltose / 14.7 mg/ml sodium citrate; (2) 50 mg/ml trehalose / 14.7 mg/ml sodium citrate and (3) 50 mg/ml sucrose 14.7 mg/ml sodium citrate. All three solutions also contained 0.2 mg/ml Polysorbate 20, NF. The three groups of solutions were freeze-dried, and the resulting lyophilisate allowed to sit for four weeks at 60°C. The lyophilisates were then tested for activity (as determined by EIA) with the following results:

| Compound: | (1) | (2) | (3) |
|---|---|---|---|
| Percentage activity: | 40% | 89% | 87% |

This EXAMPLE shows that non-reducing disaccharides aid stability better than reducing disaccharides.

### B. Stabilization of rFSH with Sodium Citrate

Two lyophilized samples are made. The first sample contains 75 Units rFSH, 25 mg amorphic sucrose, 7.35 mg sodium citrate, and 0.1 mg Polysorbate (Tween®) 20. The second sample contains 75 Units rFSH, 25 mg amorphic sucrose, and 0.2 mg Tween® 20. The pH of both samples was adjusted to 7. The first sample is stored for 3 months at 50° C, reconstituted with purified water, and analyzed by HPSEC. The resulting profile showed little oligomer formation. The second sample, not containing sodium citrate, was stored for 6 months at 50° C, reconstituted with purified water, and analyzed by HPSEC. The resulting profile showed much more oligomer formation.

The profile of the first sample showed no degradation products while the profile of the second sample showed almost exclusively oligomeric products.

## EXAMPLES II-V

### Other dicarboxylic acid salt stabilizers.

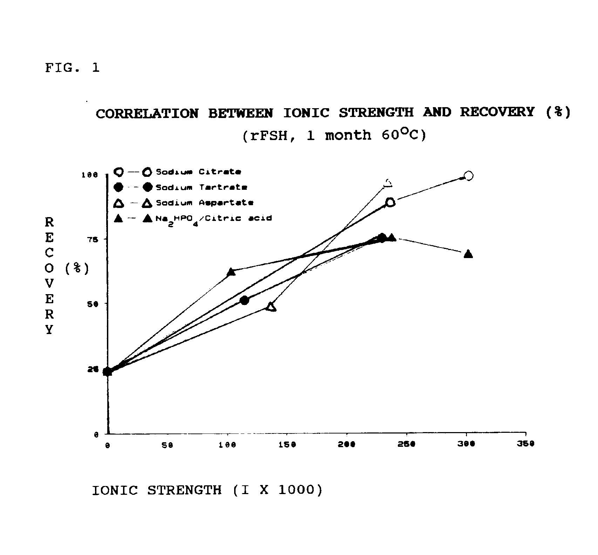

As depicted in FIG. 1, samples containing rFSH and various stabilizers were made, lyophilized, and tested for activity after 1 month storage at 60° C. FIG. 1 depicts the correlation between ionic strength (X 1000) of the particular stabilizer versus the percentage recovery (as determined by EIA).

The stabilizers tested were sodium citrate, both separately (II) and with 3.0 to 9.1 mg $Na_2HPO_4$ per ampule (III); sodium tartrate (IV); and sodium aspartate (V). Concentrations were determined in terms of ionic strength as shown in FIG. 1.

EXAMPLE VI

The effect of weight ratio of citrate to gonadotropin (HCG) activity was tested. The results are shown in FIG. 2.

EXAMPLE VII

A lyophilised composition for recombinant human FSH was made containing 75 IU rFSH, 14.7 mg sodium citrate, 50 mg sucrose, and 0.2 mg polysorbate 20. The preparation is reconstituted with one ml of water for injection.

EXAMPLE VIII

A lyophilised composition for recombinant human FSH was made containing 75 IU rFSH, 75 IU LH, 15 mg sodium citrate, 50 mg sucrose, and 0.2 mg polysorbate 20. The composition is stable. The preparation is reconstituted with one ml of water for injection.

## Claims

### Claims for the following Contracting States : AT, BE, CH, DE, DK, FR, GB, IT, LI, NL, SE

1. A stabilized gonadotropin-containing lyophilisate comprising a gonadotropin and a stabilizing amount of a dicarboxylic acid salt.

2. The stabilized gonadotropin-containing lyophilisate of claim 1 further comprising a non-reducing sugar, wherein the non-reducing sugar is present in an amount three to fifty times that by weight of the dicarboxylic acid salt stabilizer.

3. The stabilized gonadotropin-containing lyophilisate of claim 2 wherein the non-reducing sugar is present in an amount three times that by weight of the dicarboxylic acid salt stabilizer.

4. The stable gonadotropin-containing lyophilisate of any one of claims 1-3 further comprising a second gonadotropin capable of stabilization by said dicarboxylic acid salt stabilizer.

5. The stabilized gonadotropin-containing lyophilisate of any one of claims 1-4 further comprising a nonionic surfactant.

6. The stable lyophilisate of any one of claims 1-5 wherein said gonadotropin is selected from luteinizing hormone, human chorionic gonadotropin, follicle stimulating hormone, or mixtures thereof.

7. The stable gonadotropin-containing lyophilisate of any one of claims 1-6 wherein said dicarboxylic acid salt stabilizer is a salt of citric acid, tartaric acid or aspartic acid.

8. A stabilized gonadotropin-containing lyophilisate comprising:
one part by weight of a gonadotropin; and 200 to 10,000 parts by weight of a dicarboxylic acid salt stabilizer associated with said gonadotropin.

9. A method of making a stabilized gonadotropin-containing lyophilisate of claim 1 comprising: admixing, in an aqueous solution, at least one gonadotropin with an amount of dicarboxylic acid salt to adjust the ionic strength of said solution to between 0.050 and 0.350, dissolving a non-reducing sugar in said mixture in an amount of about three to fifty times the amount, by weight, of the dicarboxylic acid salt, and freeze-drying the said mixture at a temperature greater than -35° centigrade to form said stabilized gonadotropin lyophilisate.

**Claims for the following Contracting States : ES, GR**

1. A method for preparing a stabilized gonadotropin-containing lyophilisate comprising a gonadotropin and a stabilizing amount of a dicarboxylic acid salt, characterized by mixing of the gonadotropin with a sufficient amount of the dicarboxylic acid salt to stabilize the protein in the lyophilized form, and then freeze-drying the resulting solution to form a stabilized lyophilisate of the gonadotropin.

2. The method according to claim 1, wherein a non-reducing sugar is added to the mixture of the gonadotropin and the dicarboxylic acid salt in an amount three to fifty times that by weight of the dicarboxylic acid stabilizer.

3. The method according to claim 2, wherein the non-reducing sugar is added in an amount three times that by weight of the dicarboxylic acid stabilizer.

4. The method according to any one of claims 1-3, further comprising :
adding to the mixture of the gonadotropin and the dicarboxylic acid salt a second gonadotropin capable of stabilization by said dicarboxylic acid salt stabilizer.

5. The method according to any one of claims 1-4, further comprising: adding to the mixture of the gonadotropin and the dicarboxylic acid salt a nonionic surfactant.

6. The method according to any one of claims 1-5, wherein as gonadotropin is used luteinizing hormone, human chorionic gonadotropin, follicle stimulating hormone, or mixtures thereof.

7. The method according to any one of claims 1-6, wherein as dicarboxylic acid salt is used a salt of citric acid, tartaric acid or aspartic acid.

8. A method for preparing a stabilized gonadotropin-containing lyophilisate comprising a gonadotropin and a stabilizing amount of a dicarboxylic acid salt, characterized by mixing one part by weight of the gonadotropin with 200 to 10,000 parts by weight of the dicarboxylic acid salt stabilizer associated with said gonadotropin, and then freeze-drying the resulting solution to form a stabilized lyophilisate of the gonadotropin.

9. A method for preparing a stabilized gonadotropin- containing lyophilisate comprising a gonadotropin and a stabilizing amount of a dicarboxylic acid salt, characterized by admixing, in an aqueous solution, at least one gonadotropin with an amount of dicarboxylic acid salt to adjust the ionic strenght of said solution to between 0.050 and 0.350;
dissolving a non-reducing sugar in said mixture in an amount of about three to fifty times the amount, by weight, of the dicarboxylic acid salt; and
freeze-drying said mixture at a temperature greater than -35° centigrade to form said stabilized gonadotropin lyophilisate.

**Patentansprüche**

**Patentansprüche für folgende Vertragsstaaten : BE, CH, DE, FR, GB, IT, LI, LU, NL, SE**

1. Ein stabilisiertes Gonadotropin enthaltendes Lyophilisat, welches ein Gonadotropin und eine stabilisierende Menge eines Dicarbonsäuresalzes umfasst.

2. Das stabilisierte Gonadotropin enthaltende Lyophilisat gemäss Anspruch 1, welches weiterhin einen nicht-reduzierenden Zucker umfasst, wobei der nicht-reduzierende Zucker in einem drei- bis fünfzigfachen Gewichtsanteil des Dicarbonsäuresalz-Stabilisators vorhanden ist.

3. Das stabilisierte Gonadotropin enthaltende Lyophilisat gemäss Anspruch 2, worin der nicht-reduzierende Zucker in einem dreifachen Gewichtsanteil des Dicarbonsäuresalz-Stabilisators vorhanden ist.

4. Das stabile Gonadotropin-enthaltende Lyophilisat gemäss einem der Ansprüche 1 - 3, welches weiterhin ein zweites, zur Stabilisierung durch besagten Dicarbonsäuresalz-Stabilisator fähiges, Gonadotropin um-

fasst.

5. Das stabilisierte Gonadotropin enthaltende Lyophilisat gemäss einem der Ansprüche 1 - 4, welches weiterhin ein nichtionogenes Tensid enthält.

6. Das stabile Lyophilisat gemäss einem der Ansprüche 1 - 5, worin besagtes Gonadotropin aus luteotropen Hormon, menschlichem Choriogonadotropin, follikelstimulierendem Hormon oder Mischungen davon ausgewählt ist.

7. Das stabile Gonadotropin enthaltende Lyophilisat gemäss einem der Ansprüche 1 - 6, worin besagter Dicarbonsäuresalz-Stabilisator ein Salz der Zitronensäure, Weinsäure oder Asparaginsäure ist.

8. Ein stabilisiertes Gonadotropin enthaltendes Lyophilisat, umfassend:
einen Gewichtsteil Gonadotropin und 200 bis 10'000 Gewichtsteile eines mit besagtem Gonadotropin assoziierten Dicarbonsäuresalz-Stabilisators.

9. Ein Verfahren zur Herstellung eines stabilisierten Gonadotropin enthaltenden Lyophilisats gemäss Anspruch 1, umfassend:
Zumischen von mindestens einem Gonadotropin zu einer Menge Dicarbonsäuresalz in wässriger Lösung, um die Ionenstärke besagter Lösung zwischen 0,050 und 0,350 einzustellen, Lösen eines nicht-reduzierenden Zuckers, in einer Menge entsprechend der drei- bis fünfzigfachen Gewichtsmenge des Dicarbonsäuresalzes in besagter Mischung und Gefriertrocknen der besagten Mischung bei einer Temperatur höher als -35°C, um besagtes stabilisiertes Gonadotropin-Lyophilisat zu bilden.

## Patentansprüche für folgende Vertragsstaaten : ES, GR

1. Verfahren zur Herstellung eines stabilisiertes Gonadotropin enthaltenden Lyophilisats, welches ein Gonadotropin und eine stabilisierende Menge eines Dicarbonsäuresalzes umfasst, dadurch gekennzeichnet, dass das Gonadotropin mit einer ausreichenden Menge des Dicarbonsäuresalzes gemischt wird, um das Protein in der lyophilisierten Form zu stabilisieren und anschliessendem Gefriertrocknen der resultierenden Lösung, um ein stabilisiertes Lyophilisat des Gonadotropins zu bilden.

2. Das Verfahren gemäss Anspruch 1, worin ein nicht-reduzierender Zucker in einem drei- bis fünfzigfachen Gewichtsanteil des Dicarbonsäuresalz-Stabilisators zu der Mischung aus dem Gonadotropin und dem Dicarbonsäuresalz gegeben wird.

3. Das Verfahren gemäss Anspruch 2, worin der nicht-reduzierende Zucker in einem dreifachen Gewichtsanteil des Dicarbonsäuresalz-Stabilisators zugegeben wird.

4. Das Verfahren gemäss einem der Ansprüche 1 - 3, weiterhin umfassend:
Zugabe eines zweiten Gonadotropins zu der Mischung aus dem Gonadotropin und dem Dicarbonsäuresalz, welches zur Stabilisierung durch besagten Dicarbonsäuresalz-Stabilisator fähig ist.

5. Das Verfahren gemäss einem der Ansprüche 1 - 4, weiterhin umfassend:
Zugabe eines nicht-ionischen Tensids zu einer Mischung aus dem Gonadotropin und dem Dicarbonsäuresalz.

6. Das Verfahren gemäss einem der Ansprüche 1 - 5, worin luteotropes Hormon, menschliches Choriogonadotropin, follikelstimulierendes Hormon oder Mischungen davon als Gonadotropin verwendet werden.

7. Das Verfahren gemäss einem der Ansprüche 1 - 6, worin als Dicarbonsäuresalz ein Salz der Zitronensäure, Weinsäure oder Asparaginsäure verwendet wird.

8. Verfahren zur Herstellung eines stabilisiertes Gonadotropin enthaltenden Lyophilisats, welches ein Gonadotropin und eine stabilisierende Menge eines Dicarbonsäuresalzes umfasst, dadurch gekennzeichnet, dass ein Gewichtsanteil des Gonadotropins mit 200 bis 10'000 Gewichtsanteilen des mit besagtem Gonadotropin assoziierten Dicarbonsäuresalz-Stabilisators gemischt wird und anschliessendem Gefriertrocknen der resultierenden Lösung, um ein stabilisiertes Lyophilisat des Gonadotropins zu bilden.

9. Verfahren zur Herstellung eines stabilisiertes Gonadotropin enthaltenden Lyophilisats, welches ein Gonadotropin und eine stabilisierende Menge eines Dicarbonsäuresalzes umfasst, gekennzeichnet durch Zumischen von mindestens einem Gonadotropin zu einer Menge Dicarbonsäuresalz in wässriger Lösung, um die Ionenstärke besagter Lösung zwischen 0,050 und 0,350 einzustellen; Lösen einer Menge eines nicht-reduzierenden Zuckers in besagter Mischung, welche ungefähr der drei- bis fünfzigfachen Menge des Gewichtsanteils des Dicarbonsäuresalzes entspricht und Gefriertrocknen besagter Mischung bei einer Temperatur höher als -35°C, um besagtes stabilisiertes Gonadotropin-Lyophilisat zu bilden.

## Revendications

### Revendications pour les Etats contractants suivants : AT, BE, CH, DE, FR, GB, IT, LI, LU, NL, SE, DK

1. Un lyophilisat contenant de la gonadotrophine stabilisée comprenant de la gonadotrophine et une quantité stabilisante d'un sel d'acide dicarboxylique.

2. Le lyophilisat contenant une gonadotrophine stabilisée selon la revendication 1, comprenant de plus un sucre non réducteur dans lequel le sucre non réducteur est présent en une quantité pondérale représentant 3 à 50 fois la quantité d'agent stabilisant à base de sel d'acide dicarboxylique.

3. Le lyophilisat contenant de la gonadotrophine stabilisée selon la revendication 2, caractérisé en ce que le sucre non réducteur est présent en une quantité pondérale représentant 3 fois la quantité d'agent stabilisant à base de sel d'acide dicarboxylique.

4. Le lyophilisat contenant de la gonadotrophine stabilisée selon l'une quelconque des revendications 1 à 3, comprenant en outre une seconde gonadotrophine capable d'être stabilisée par lesdits agents stabilisants à base de sels d'acide dicarboxylique.

5. Le lyophilisat contenant de la gonadotrophine stabilisée selon l'une quelconque des revendications 1 à 4, comprenant en outre un tensioactif non ionique.

6. Le lyophilisat stable selon l'une quelconque des revendications 1 à 5, caractérisé en ce que ladite gonadotrophine est sélectionnée parmi la lutéinostimuline, la gonadotrophine chorionique humaine, la folliculostimuline ou leurs mélanges.

7. Le lyophilisat contenant de la gonadotrophine stable selon l'une quelconque des revendications 1 à 6, caractérisé en ce que ledit agent stabilisant à base de sel d'acide dicarboxylique correspond à un sel d'acide citrique, d'acide tartrique ou d'acide aspartique.

8. Un lyophilisat contenant de la gonadotrophine stabilisée comprenant:
   - une partie en poids d'une gonadotrophine; et
   - de 200 à 10000 parties en poids d'un agent stabilisant constitué d'un sel d'acide dicarboxylique associé à ladite gonadotrophine.

9. Un procédé de préparation d'un lyophilisat contenant de la gonadotrophine stabilisée selon la revendication 1, comprenant:
   - le mélange en solution aqueuse d'au moins une gonadotrophine avec une quantité d'un sel d'acide carboxylique pour ajuster la force ionique de ladite solution à une valeur de 0,050 à 0,350;
   - la dissolution d'un sucre non réducteur dans ledit mélange en une quantité représentant environ 3 à 50 fois la quantité pondérale du sel d'acide dicarboxylique; et
   - la lyophilisation dudit mélange à une température supérieure à -35°C pour former ledit lyophilisat de gonadotrophine stabilisée.

### Revendications pour les Etats contractants suivants : ES, GR

1. Un procédé de préparation d'un lyophilisat contenant de la gonadotrophine stabilisée comprenant de la gonadotrophine et une quantité stabilisante d'un sel d'acide dicarboxylique, caractérisé en ce que la gonadotrophine est melangé avec une quantité suffisante d'un sel d'acide dicarboxylique pour stabiliser la protéine sous la forme lyophilisée, puis la solution résultante est lyophilisée pour former un lyophilisat stabilisé de la gonadotrophine.

2. Le procédé selon la revendication 1, dans lequel un sucre non réducteur est ajouté a le mélange de la gonadotrophine et la sel d'acide dicarboxylique en une quantité pondérale représantant 3 à 50 fois la quantité d'agent stabilisant à base d'acide dicarboxylique.

3. Le procédé selon la revendication 2, dans lequel le sucre non réducteur est ajouté en une quantité pondérale 3 fois la quantité d'agent stabilisant à base d'acide dicarboxylique.

4. Le procédé selon l'une quelconque des revendications 1 à 3, comprenant en outre:
ajouter à la mélange de la gonadotrophine et la sel d'acide dicarboxylique une seconde gonadotrophine capable d'être stabilisée par lesdits agents stabilisants à base de sels d'acide dicarboxylique.

5. Le procédé selon l'une quelconque des revendications 1 à 4, comprenant en outre:
ajouter à la mélange de la gonadotrophine et la sel d'acide dicarboxylique un tensioactif non ionique.

6. Le procédé selon l'une quelconque des revendications 1 à 5, dans lequel la gonadotrophine utilisée est sélectionnée parmi la lutéinostimuline, la gonadotrophine chorionique humaine, la folliculostimuline ou lears mélanges.

7. Le procédé selon l'une quelconque des revendications 1 à 6, dans lequel l'agent stabilisant à base de sel d'acide dicarboxylique utilisé est selectionnée parmi un sel d'acide citrique, d'acide tartrique ou d'acide aspartique.

8. Un procédé de préparation d'un lyophilisat contenant de la gonadotrophine stabilisée comprenant de la gonadotrophine et une quantité stabilisante d'un sel d'acide dicarboxylique, caractérisé en ce que une partie en poids d'une gonadotrophine est melangée avec 200 à 10,000 parties en poids d'un agents stabilisant constitué d'un sel d'acide dicarboxylique associé à ladite gonadotrophine, et puis la solution résultante est lyophilisée pour former un lyophilisat stabilisé de la gonadotrophine.

9. Un procédé de préparation d'un lyophilisat contenant de la gonadotrophine stabilisée comprenant de la gonadotrophine et une quantité stabilisante d'un sel d'acide dicarboxylique, comprenant:
le mélange en solution aqueuse d'au moins une gonadotrophine avec une quantité d'un sel d'acide carboxylique pour ajuster la force ionique de ladite solution à une valeur de 0,050 à 0,350;
la dissolution d'un sucre non réducteur dans ledit mélange en une quantité représantant environ 3 à 50 fois la quantité pondérale du sel d'acide dicarboxylique; et
la lyophilisation dudit mélange à une température supérieure à -35 °C pour former ledit lyophilisat de gonadotrophine stabilisée.

FIG. 1

CORRELATION BETWEEN IONIC STRENGTH AND RECOVERY (%)

(rFSH, 1 month 60°C)

IONIC STRENGTH (I X 1000)

FIG. 2

Effect of citrate/protein ratio on recovery of hCG activity

recovery as % of activity in solution

ratio citrate/protein (mg/mg)

○ recovery after freeze-drying
● recovery after storage at 50C